# EUROPEAN PATENT SPECIFICATION

(11) **EP 1 810 699 B1**
(45) Date of publication and mention of the grant of the patent: **23.03.2011**
(21) Application number: 07001213.3
(22) Date of filing: 19.01.2007
(51) Int. Cl.: A61L 26/00

(54) **Biodegradable hemostatic compositions**
Biologisch abbaubare hämostatische Zusammensetzungen
Compositions hémostatiques biodégradables

(30) Priority: 23.01.2006 US 761128 P
(43) Date of publication of application: 25.07.2007
(73) Proprietor: Tyco Healthcare Group LP, New Haven, CT 06511 (US)
(72) Inventor: Stopek, Joshua, Yalesville, CT 06492 (US)
(74) Representative: HOFFMANN EITLE

(56) References cited:
- EP-A- 1 391 205
- US-A- 6 162 241
- DATABASE WPI Week 200241 Derwent Publications Ltd., London, GB; AN 2002-377469 XP002448618 & JP 2002 060341 A (TERUMO CORP) 26 February 2002 (2002-02-26)

## Description

### TECHNICAL FIELD

The present disclosure relates to biodegradable hemostatic compositions capable of forming a matrix and the use of these polymers as hemostatic agents or sealants.

### BACKGROUND

The control of bleeding both during and after a surgical procedure is of great importance. Hemostasis, a term of art which refers to cessation of bleeding, may frequently be accomplished through the use of mechanical devices such as sutures, staples, and the like, as well as chemical and/or biological compositions which assist in stopping bleeding.

For hemostatic compositions, many clinicians have focused on the development of compositions which include various coagulation factors and function by exploiting the body's own hemostatic processes to promote rapid hemostasis and wound healing. Suitable hemostatic agents should exhibit high initial tack and an ability to bond rapidly to living tissue; the strength of the bond should be sufficiently high to cause tissue failure before bond failure; the hemostatic agent should form a bridge, typically a permeable flexible bridge; and the bridge and/or its metabolic products should not cause local histotoxic or carcinogenic effects.

Several materials useful as hemostatic agents are currently available. One type of hemostatic agent that is currently available is a cyanoacrylate adhesive. However, there is the possibility that a cyanoacrylate adhesive can degrade and generate undesirable by-products such as formaldehyde. Another disadvantage with cyanoacrylate adhesives is that they can have a high flexural modulus which can limit their usefulness.

Another type of hemostatic agent that is currently available utilizes components derived from bovine and/or human sources. For example, thrombin-based formulations for use as hemostatics are also known. However, the use of thrombin in hemostatic formulations is limited by its instability during storage.

Recent developments have also led to the production of thrombin-fibrin compositions, which are sometimes referred to as "fibrin glues". The thrombin functions as the "catalyst" component of the glue, and the fibrin functions as the "resin" component of the glue. However, as with any natural material, variability in the material is frequently observed and, because the hemostatic agent is derived from natural proteins, there may be viral transmission concerns.

EP 1 391 205 discloses pharmaceutical compositions for enhancing blood clotting that comprise RNA as a cofactor for initiating the contact phase system of blood coagulation.

JP 2002060341 discloses a haemostatic agent containing calcium salts of nucleic acids, in particular in combination with protamine, collagen or gelatine.

US 6,162,241 discloses a method of controlling hemostasis by applying a hemostatic agent in a tissue sealant composition. The tissue sealant is a biodegradable, biocompatible synthetic polymer with a haemostat incorporated therein. The composition may further comprise small nucleotides.

It would be desirable to provide a biocompatible composition that is suitable for use as a hemostatic agent or sealant. Such a hemostatic agent should be non-inflammatory and not transmit infectious diseases.

### SUMMARY

The present disclosure provides biocompatible hemostatic compositions which include at least one nucleic acid. The nucleic acid is biodegradable and non-inflammatory and may be obtained from plant sources or animal sources. In other embodiments the nucleic acid may be synthetic. In embodiments, the nucleic acid may include ribonucleotides or deoxyribonucleotides. In some embodiments, the nucleic acid may be modified.

The compositions of the present disclosure may also include medicinal agents and/or enzymes.

The compositions of the present disclosure may be sprayable. In other embodiments, the compositions of the present disclosure may be a film or a foam.

Methods for promoting hemostasis of a tissue site in an animal are also provided whereby the tissue site is contacted with a hemostatic composition of the present disclosure.

### DETAILED DESCRIPTION

The present disclosure relates to hemostatic compositions which are biocompatible, non-inflammatory, and biodegradable. The biodegradable hemostatic composition can be employed to seal fluid leaks in tissues, and is especially adapted to assist in hemostasis. The biodegradable hemostatic composition of the present disclosure can be applied to living tissue and/or flesh of animals, including humans.

Unless defined otherwise, all technical and scientific terms used herein have the same meaning as commonly understood by one of ordinary skill in the art to which this disclosure belongs.

While certain distinctions may be drawn between the usage of the terms "flesh" and "tissue" within the scientific community, the terms are used interchangeably herein as referring to a general substrate upon which those skilled in the art would understand the present hemostatic composition to be utilized within the medical field for the treatment of patients. As used herein, "tissue" may include, but is not limited to, skin, bone, neuron, axon, cartilage, blood vessel, cornea, muscle, fascia, brain, prostate, breast, endometrium, lung, pancreas, small intestine, blood, liver, testes, ovaries, cervix, colon, stomach, esophagus, spleen, lymph node, bone marrow, kidney, peripheral blood, embryonic or ascite tissue.

In accordance with the present disclosure, biodegradable hemostatic compositions are provided wherein the biodegradable hemostatic composition is a matrix of nucleic acids.

The terms "nucleic acid" and "nucleic acid molecule" are used interchangeably herein and refer to a molecule including nucleotides, i.e., ribonucleotides, deoxyribonucleotides, or both. The terms include monomers, oligomers and polymers, i.e., polynucleotides, of ribonucleotides and/or deoxyribonucleotides. The ribonucleotides and/or deoxyribonucleotides may be connected together, in the case of polymers, via 5' to 3' linkages. In addition, polymers may be joined by any other linkages within the purview of one skilled in the art including, for example, nucleic acids having 5' to 2' linkages.

The term "monomer" as used herein refers to a nucleic acid molecule or derivative thereof containing a single nucleotide.

The term "oligonucleotide" as used herein refers to a nucleic acid molecule including from about 2 to about 100 nucleotides, in embodiments from about 3 to about 80 nucleotides, typically from about 4 to about 35 nucleotides.

The term "polynucleotide" as used herein refers to a nucleic acid molecule including at least three nucleotides, typically more than about 10 nucleotides, in embodiments from about 100 nucleotides to about 100,000 nucleotides, in other embodiments from about 500 nucleotides to about 50,000 nucleotides. DNAs and RNAs are examples of polynucleotides.

The nucleotides used in the nucleic acid molecule may be naturally occurring or may be synthetically produced analogues that are capable of forming base-pair relationships with naturally occurring base pairs.

Suitable natural sources of nucleic acids for use in accordance with the present disclosure include both plant and animal sources, as well as combinations thereof. Particularly useful plant sources for nucleic acids suitable for use in compositions of the present disclosure include onions, broccoli, tomatoes, and the like. Particularly useful animal sources for nucleic acids in accordance with the present disclosure include human, bovine, equine, murine, and caprine. The nucleic acids utilized to form the biodegradable hemostatic composition of the present disclosure may be from the same plant species, the same animal species, a mixture of nucleic acids from different plant species, a mixture of nucleic acids from different animal species, or any combination thereof.

In another embodiment, the nucleic acids utilized in the present disclosure may be synthetic. Synthetic nucleic acids may be produced utilizing methods within the purview of those skilled in the art and, in some embodiments, may utilize non-naturally occurring bases capable of forming base-pairing relationships. Examples of non-naturally occurring bases that are capable of forming base-pairing relationships and may be utilized to form synthetic nucleic acids include, but are not limited to, aza and deaza pyrimidine analogues, aza and deaza purine analogues, and other heterocyclic base analogues, wherein one or more of the carbon and nitrogen atoms of the purine and pyrimidine rings have been substituted by heteroatoms, e.g., oxygen, sulfur, selenium, phosphorus, etc.

Methods for synthesizing nucleic acids, including oligonucleotides and polynucleotides, are within the purview of those skilled in the art and include, for example, direct chemical synthesis by methods such as the phosphotriester method of Narang et al., Meth. Enzymol. 68:90-99 (1979); the phosphodiester method of Brown et al., Meth. Enzymol. 68:109-151 (1979); the diethylphosphoramidite method of Beaucage et al., Tetra. Lett. 22:1859-1862 (1981); the solid phase phosphoramidite triester method described by Beaucage and Caruthers, Tetra. Letts. 22(20):1859-1862 (1981); using an automated synthesizer, e.g., as described in Needham-VanDevanter et al., Nucleic Acids Res., 12:6159-6168 (1984), or on commercially purchased DNA synthesizers from <1 uM to >1 mM scales using standard phosphoramide chemistry and methods that are disclosed in Stec et al., 1984, J. Am. Chem. Soc. 106:6077-6089, Stec et al., 1985, J Org. Chem. 50(20):3908-3913, Stec et al., 1985, J. Chromatog. 326:263-280, LaPlanche et al., 1986, Nuc. Acid. Res. 14(22):9081-9093, and Fasman, 1989, Practical Handbook of Biochemistry and Molecular Biology, 1989, CRC Press, Boca Raton, Fla.; and the solid support method of U.S. Pat. No. 4,458,066.

In some embodiments, where chemical synthesis produces a single stranded nucleic acid, especially an oligonucleotide, this oligonucleotide may be converted into double stranded DNA by hybridization with a complementary sequence, or by polymerization with a DNA polymerase using the single strand as a template. As will be readily apparent to one skilled in the art, while chemical synthesis of DNA is limited to sequences of about 100 bases, longer sequences may be obtained by the ligation of shorter sequences.

Synthetic nucleic acids utilized herein to form the biodegradable hemostatic composition of the present disclosure may also include natural nucleosides (i.e., adenosine, thymidine, guanosine, cytidine, uridine, deoxyadenosine, deoxythymidine, deoxyguanosine, and deoxycytidine), nucleoside analogs (e.g., 2-aminoadenosine, 2-thiothymidine, inosine, pyrrolo-pyrimidine, 3-methyl adenosine, C5-propynylcytidine, C5-propynyluridine, C5-bromouridine, C5-fluorouridine, C5-iodouridine, C5-methylcytidine, 7-deazaadenosine, 7-deazaguanosine, 8-oxoadenosine, 8-oxoguanosine, 0(6)-methylguanine, and 2-thiocytidine), chemically modified bases, biologically modified bases (e.g., methylated bases), intercalated bases, modified sugars (e.g., 2'-fluororibose, ribose, 2'-deoxyribose, arabinose, and hexose), or modified phosphate groups (e.g., phosphorothioates and 5'-N-phosphoramide linkages).

In some embodiments, the nucleic acid utilized to form the hemostatic composition of the present disclosure may be modified. The term "modified nucleic acid" as used herein includes "modified oligonucleotide", "modified polynucleotide", and "modified monomer", and refers to nucleic acids with one or more chemical modifications at the molecular level. Such modifications may include, for example, modifications of all or any of the nucleic acid bases, sugar moieties, internucleoside phosphate linkages, as well as molecules having added substituents, such as diamines, cholesteryl or other lipophilic groups, or a combination of these modifications. The internucleoside phosphate linkages can be phosphodiester, phosphotriester, phosphoramidate, siloxane, carbonate, carboxymethylester, acetamidate, carbamate, thioether, bridged phosphoramidate, bridged methylene phosphonate, phosphorothioate, methylphosphonate, phosphorodithioate, bridged phosphorothioate and/or sulfone internucleotide linkages, or 3'-3', 2'-5', or 5'-5' linkages, and combinations of such similar linkages to produce mixed backbone modified oligonucleotides.

Methods for modifying the nucleic acids of the present disclosure, e.g., monomers, oligonucleotides, polynucleotides, etc., are within the purview of those skilled in the art. For example, modifications to bases utilized in the hemostatic compositions herein can be internal (single or repeated) or at the end(s) of the nucleic acid molecule, particularly in the case of oligonucleotides and polynucleotides. Such modifications can include additions to the internucleoside phosphate linkages, such as cholesteryl; diamine compounds with varying numbers of carbon residues between amino groups and terminal ribose; and deoxyribose and phosphate modifications which cleave or cross-link to the opposite chains or to associated enzymes formed or other proteins. Electrophilic groups such as ribose-dialdehyde could be formed which covalently link with an epsilon amino group of the lysyl-residue of a protein. A nucleophilic group such as n-ethylmaleimide tethered to an oligomer could covalently attach to the 5' end of an mRNA or to another electrophilic site.

Moreover, should the polynucleotide have a molecular weight that is deemed too large for compositions of the present disclosure, said polynucleotide may be degraded to an appropriate size utilizing techniques within the purview of those skilled in the art. Such degradation techniques include, but are not limited to, enzymatic treatments, chemical treatments, oxidation, radiation treatments, thermal treatments, and the like.

The term "modified oligonucleotides" also includes oligonucleotides having modifications to the sugar moieties such as 2'-substituted ribonucleotides, or deoxyribonucleotide monomers, any of which are connected together via 5' to 3' linkages. Modified oligonucleotides may also include PNA or morpholino modified backbones.

Once obtained, the nucleic acids of the present disclosure may be cloned and/or amplified utilizing standard recombinant techniques such as polymerase chain reaction (PCR). A polynucleotide of the present disclosure can be attached to a vector, adapter, promoter, transit peptide or linker for cloning of a polynucleotide of the present disclosure. Additional sequences may be added to such cloning expression sequences to optimize their function in cloning, and/or to aid in isolation of the polynucleotide. Use of cloning vectors, adapters, and linkers is extensively described and within the purview of those skilled in the art. For a description of such nucleic acids see, for example, Stratagene Cloning Systems, Catalogs 1995, 1996, 1997 (La Jolla, Calif.); and, Amersham Life Sciences, Inc, Catalog '97 (Arlington Heights, III.).

The molecular weight of the resulting nucleic acids for use in the hemostatic compositions of the present disclosure can encompass a wide range, depending on whether the nucleic acids are monomers, oligonucleotides, or polynucleotides. Obviously, lower ranges will more likely encompass the use of monomers, while higher ranges will encompass the use of oligonucleotides/polynucleotides.

Synthetic nucleic acids utilized in compositions of the present disclosure may be used alone, combined with different synthetic nucleic acids, combined with nucleic acids from plants, combined with nucleic acids from animals, or combinations thereof.

In some embodiments, it may be desirable to purify the nucleic acids utilized to produce the biodegradable hemostatic compositions of the present disclosure. A variety of standard methods within the purview of those skilled in the art may be used to purify the presently described nucleic acids. In brief, the nucleic acids of the present disclosure can be purified by chromatography on commercially available reverse phase media (for example, see the RAININ Instrument Co., Inc. instruction manual for the DYNAMAX®-300A, Pure-DNA reverse phase columns (1989), or current updates thereof) or ion exchange media such as Waters' Protein Pak or Pharmacia's Source Q (see generally Warren and Vella, 1994, "Analysis and Purification of Synthetic Nucleic Acids by High-Performance Liquid Chromatography", in Methods in Molecular Biology, vol. 26; Protocols for Nucleic Acid Conjugates, S. Agrawal, ed. Humana Press, Inc., Totowa, N.J.; Aharon et al., 1993, J. Chrom. 698:293-301; and Millipore Technical Bulletin, 1992, Antisense DNA: Synthesis, Purification, and Analysis). Peak fractions can be combined and the samples concentrated and desalted via alcohol (ethanol, butanol, isopropanol, and isomers and mixtures thereof, etc.) precipitation, reverse phase chromatography, diafiltration, or gel filtration.

In embodiments, the nucleic acids of the present disclosure may be purified by chromatography on commercially available reverse phase or ion exchange media, e.g., WATERS PROTEIN-PAK^{™}, Pharmacia's SOURCE Q, etc. Peak fractions can be combined and the samples desalted and concentrated by means of reverse phase chromatography on a poly(styrenedivinylbenzene) based media, such as Hamilton's PRP1 or PRP3, or Polymer Labs' PLRP resins. Alternatively, ethanol precipitation, diafiltration, or gel filtration may be used followed by lyophilization or solvent evaporation under vacuum in commercially available instrumentation such as Savant's SPEED VAC^{®}. Optionally, small amounts of the nucleic acids may be electrophoretically purified using polyacrylamide gels.

A nucleic acid or polynucleic acid is considered pure when it has been isolated so as to be substantially free of, inter alia, contaminants which may hinder or otherwise interfere with the ability of the oligonucleotide or polynucleotide to form a biodegradable hemostatic composition.

As noted above, in some embodiments the nucleic acids may be completely or partially derivatized/modified by a chemical moeity including, but not limited to, phosphodiester linkages, phosphotriester linkages, phosphoramidate linkages, siloxane linkages, carbonate linkages, carboxymethylester linkages, acetamidate linkages, carbamate linkages, thioether linkages, bridged phosphoramidate linkages, bridged methylene phosphonate linkages, phosphorothioate linkages, methylphosphonate linkages, phosphorodithioate linkages, morpholino, bridged phosphorothioate linkages, sulfone internucleotide linkages, 3'-3' linkages, 5'-2' linkages, 5'-5' linkages, 2'-deoxy-erythropentofuranosyl, 2'-fluoro, 2'-O-alkyl nucleotides, 2'-O-alkyl-n(O-alkyl) phosphodiesters, morpholino linkages, p-ethoxy oligonucleotides, PNA linkages, p-isopropyl oligonucleotides, or phosphoramidates.

Once obtained, the nucleic acids may be combined to form a biodegradable, non-inflammatory hemostatic composition of the present disclosure. Such a hemostatic composition may be applied to a tissue site in an animal, especially a site at which hemostasis needs to be restored. The nucleic acids may be applied to a tissue site and allowed to form a matrix in situ, or the nucleic acids may first be formed into a film or foam ex vivo, utilizing methods within the purview of those skilled in the art, and then applied to the tissue site to restore hemostasis.

Depending on the nucleic acid utilized, the biodegradable hemostatic compositions of the present disclosure can possess varied morphologies, i.e., nano-macroporous, interconnected, closed-structured, channeled, ordered, random, nano-micropatterned, templated, and the like.

The concentrations of the nucleic acids utilized to produce the hemostatic compositions of the present disclosure will vary depending upon a number of factors, including the types and molecular weights of the particular nucleic acids used and the desired end use application, i.e., as a spray, film or foam. The use of higher concentrations of the nucleic acids will result in the formation of a more tightly crosslinked biodegradable hemostatic composition, producing a stiffer and stronger gel matrix.

In some embodiments, the nucleic acids may be cross-linked by subjecting them to treatments within the purview of those skilled in the art including, but not limited to, ionic, covalent, electrostatic, physical, thermal, and the like. Cross-linking may be accomplished in varying degrees to regulate the degree of swelling, the rate of degradation and the particle size of the compositions of the present disclosure.

The compositions of the present disclosure may also be sterilized utilizing methods within the purview of those skilled in the art including, but not limited to, gamma radiation, plasma sterilization, autoclave sterilization, ethylene oxide (EtO) sterilization, e-beam sterilization, peroxide treatments, aseptic treatments, and the like.

In some embodiments, the biodegradable hemostatic compositions of the present disclosure may be formulated for administration in an aqueous medium which may optionally contain buffers, viscosifiers, osmolality enhancers, and other substances that are desired and/or necessary to assure biocompatibility, injectability and efficacy. Depending on the particular application, the compositions can be formulated in various different configurations.

The nucleic acids of the present disclosure may also be complexed, conjugated, mixed, or loaded with medicinal agents. Medicinal agents can include synthetic polymers such as hydrogels, drugs, proteins, polysaccharides, lipids, antiviral agents, biocides, and the like. Some examples of medicinal agents which may be added to the biodegradable hemostatic composition include antimicrobial agents, colorants, preservatives, or medicinal agents such as, for example, protein and peptide preparations, antipyretic, antiphlogistic and analgesic agents, anti-inflammatory agents, vasodilators, antihypertensive and antiarrhythmic agents, hypotensive agents, antitussive agents, antineoplastics, local anesthetics, hormone preparations, antiasthmatic and antiallergic agents, antihistaminics, anticoagulants, antispasmodics, cerebral circulation and metabolism improvers, antidepressant and antianxiety agents, vitamin D preparations, hypoglycemic agents, antiulcer agents, hypnotics, antibiotics, antifungal agents, sedative agents, bronchodilator agents, and dysuric agents).

A phospholipid surfactant that provides antibacterial stabilizing properties and helps dispense other-materials in the biodegradable hemostatic composition may also be added to the hemostatic composition of the present disclosure.

Imaging agents such as iodine or barium sulfate, or fluorine, can also be combined with the biodegradable hemostatic composition of the present disclosure to allow visualization of the surgical area through the use of imaging equipment, including X-ray, MRI, and CAT scan.

Additionally, an enzyme may be added to the biodegradable hemostatic composition of the present disclosure to increase its rate of degradation. Suitable enzymes include, for example, peptide hydrolases such as elastase, cathepsin G, cathepsin E, cathepsin B, cathepsin H, cathepsin L, trypsin, pepsin, chymotrypsin, y-glutamyltransferase (γ-GTP) and the like; sugar chain hydrolases such as phosphorylase, neuraminidase, dextranase, amylase, lysozyme, oligosaccharase and the like; oligonucleotide hydrolases such as alkaline phosphatase, endoribonuclease, endodeoxyribonuclease and the like. In some embodiments, where an enzyme is added, the enzyme may be included in a liposome or microsphere to control the rate of its release, thereby controlling the rate of degradation of the biodegradable hemostatic composition of the present disclosure. Methods for incorporating enzymes into liposomes and/or microspheres are within the purview of those skilled in the art.

Application of the biodegradable, non-inflammatory hemostatic composition with or without other additives can be done by any conventional means. These include dripping, brushing, or other direct manipulation of the biodegradable hemostatic composition on the tissue surface, or spraying of the biodegradable hemostatic composition to the surface. Spraying may be accomplished with currently available spray equipment for use with biodegradable hemostatic compositions. In other embodiments, the compositions of the present disclosure may be formed into a dry film or foam and applied to tissue utilizing methods currently in use for hemostatic materials in the film or foam state. In open surgery, application by hand, forceps or the like is contemplated. In endoscopic surgery, the biodegradable hemostatic composition can be delivered through the cannula of a trocar, and spread at the site by any device within the purview of those skilled in the art. The biodegradable hemostatic composition can also be dispensed from a conventional adhesive dispenser within the purview of those skilled in the art.

The biodegradable, non-inflammatory hemostatic composition may also be applied to a substrate such as a particle, film, foam, and the like, to improve the hemostatic properties of the substrate and thereby limit the amount of nucleic acid required to achieve hemostasis. Compositions of the present disclosure may also be applied to staple lines, anastomoses, suture lines, mesh fixation sites, clip fixation sites, cut lines of instruments, ligation lines, and the like to further promote healing and hemostasis.

In embodiments, the compositions of the present disclosure can be utilized as hemostatic agents. The compositions swell on contact with blood, pulling platelets, clotting factors, and other molecules/cells to the surface of the composition to initiate clotting and rapidly induce hemostasis. Because they are natural in origin, the compositions of the present disclosure will be degraded by enzymes in situ in the host animal and excreted from the body after several days, but after hemostasis has been restored to the affected site to which the biodegradable hemostat composition of the present disclosure has been applied.

The clotting efficiency of the biodegradable hemostatic compositions can easily be determined by observing instantaneous clot formation upon administration. However, in order to compare the efficiency of clot formation of various different formulations for purposes of optimization, such comparisons can be made on the basis of rheometric measurements which are taken during clot formation. As described by Rosenblatt, et al. (J. Appl. Polym. Sci. 50: 953-963 (1993)), the dynamic elastic modulus, G', and the dynamic viscous modulus, G", are determined as a function of the elasticity and the overall strength of a gel, respectively. (See, also, Ferry, Viscoelastic Properties of Polymers, 3d ed., John Wiley, New York, pages 1-31 and 41-44 (1980); and Janmey, et al., Blood, 80(4): 928-936 (1992).)

The compositions of the present disclosure are useful in many different applications where hemostatic agents, tissue sealants and tissue adhesives are normally used. The biodegradable hemostatic compositions of the present disclosure can be used in a medical/surgical capacity in place of, or in combination with, sutures, staples, clamps and the like. The biodegradable hemostatic composition of the present disclosure can also be used in surgery to prevent or inhibit bleeding or fluid leakage both during and after a surgical procedure.

The biodegradable hemostatic composition of the present disclosure may be useful for stopping diffuse capillary bleeding, for example in parenchymal organ bleeding, such as that of the liver, spleen and/or kidney. The compositions of the present disclosure may be utilized to maintain hemostasis in procedures prone to excessive post-surgical bleeding. Such bleeding can pose problems during a variety of different surgical procedures, such as in the field of orthopedics, neurosurgery, plastic and reconstructive surgery, spinal surgery and oral-maxillo-facial surgery. Post-surgical application of the compositions of the present disclosure can therefore be used to lessen post-surgical blood loss.

The compositions of the present disclosure may also be useful in controlling intraoperative bleeding that has been exacerbated by genetic or acquired clotting defects or the use of anticoagulation therapy. For example, if a patient receives anticoagulant therapy following surgery and subsequently needs additional surgery, the compositions of the present disclosure may be useful in counteracting any increased bleeding caused by the anticoagulants.

The present biodegradable hemostatic composition has a number of advantageous properties. The resulting biodegradable hemostatic compositions of the present disclosure are safe and biocompatible, possess enhanced adherence to tissue, are biodegradable, have enhanced hemostatic potential, have low cost, and are easy to prepare and use. By varying the selection of the polymer components, the strength and elasticity of the biodegradable hemostatic composition can be controlled, as can the gelation time.

Additionally, the hemostatic compositions of the present disclosure are biodegradable, allowing the degradation components to pass safely through the subject's body.

## Claims

1. A hemostatic composition comprising a matrix of at least one biodegradable and non-inflammatory nucleic acid for inducing hemostasis by swelling in contact with blood, and pulling platelets, clotting factors and other molecules and/or cells to the surface of the composition to initiate clotting.

2. The hemostatic composition of claim 1, wherein the at least one nucleic acid is synthetic.

3. The hemostatic composition of claim 1, wherein the at least one nucleic acid is obtained from at least one plant.

4. The hemostatic composition of claim 3, wherein the at least one plant is selected from onions, broccoli and tomatoes.

5. The hemostatic composition of Claim 1, wherein the at least one nucleic acid is obtained from at least one animal.

6. The hemostatic composition of claim 5, wherein the at least one animal is selected from human, bovine, equine, murine, and caprine.

7. The hemostatic composition of any of the preceding claims, wherein the at least one nucleic acid is selected from ribonucleotides and deoxyribonucleotides.

8. The hemostatic composition of any of the preceding claims, wherein the composition has been sterilized.

9. The hemostatic composition of any of the preceding claims, which further comprises at least one medicinal agent.

10. The hemostatic composition of any of the preceding claims, which further comprises at least one enzyme for increasing the rate of degradation of the hemostatic composition.

11. The hemostatic composition of any of the preceding claims, which is present in form of a sprayable composition, a film or a foam.

12. Use of at least one biodegradable, non-inflammatory nucleic acid for the manufacture of a hemostatic composition according to any of claims 1-11 suitable for promoting hemostasis of a tissue site in an animal subject or for promoting the hemostatic properties of a substrate.

13. The use of claim 12, wherein the hemostatic composition is provided in a form for dripping, brushing, direct manipulation or spraying on a tissue surface.

14. The use of claim 12, wherein the hemostatic composition is provided in form of a film or a foam.

15. Biodegradable, non-inflammatory nucleic acid for use in the formation of a biodegradable hemostatic matrix of nucleic acids in a tissue site for inducing hemostasis by swelling in contact with blood, and pulling platelets, clotting factors and other molecules and/or cells to the surface of the composition to initiate clotting.

## Patentansprüche

1. Blutstillende Zusammensetzung, umfassend ein Grundgerüst von mindestens einer biologisch abbaubaren und nicht-inflammatorischen Nukleinsäure zum Herbeiführen der Blutstillung durch Anschwellen bei Kontakt mit Blut und Anziehen von Blutplättchen, Gerinnungsfaktoren und anderen Molekülen und/oder Zellen zur Oberfläche der Zusammensetzung, um die Blutgerinnung auszulösen.

2. Blutstillende Zusammensetzung gemäß Anspruch 1, wobei die mindestens eine Nukleinsäure synthetisch ist.

3. Blutstillende Zusammensetzung gemäß Anspruch 1, wobei die mindestens eine Nukleinsäure von mindestens einer Pflanze gewonnen wird.

4. Blutstillende Zusammensetzung gemäß Anspruch 3, wobei die mindestens eine Pflanze ausgewählt wird zwischen Zwiebeln, Broccoli und Tomaten.

5. Blutstillende Zusammensetzung gemäß Anspruch 1, wobei die mindestens eine Nukleinsäure von mindestens einem Tier gewonnen wird.

6. Blutstillende Zusammensetzung gemäß Anspruch 5, wobei das mindestens eine Tier ausgewählt wird zwischen Mensch, Rind, Pferd, Maus und Ziege.

7. Blutstillende Zusammensetzung gemäß einem beliebigen der vorhergehenden Ansprüche, wobei die mindestens eine Nukleinsäure ausgewählt wird zwischen Ribonukleotiden und Deoxyribonukleotiden.

8. Blutstillende Zusammensetzung gemäß einem beliebigen der vorhergehenden Ansprüche, wobei die Zusammensetzung sterilisiert wurde.

9. Blutstillende Zusammensetzung gemäß einem beliebigen der vorhergehenden Ansprüche, die weiterhin mindestens ein Arzneimittel umfasst.

10. Blutstillende Zusammensetzung gemäß einem beliebigen der vorhergehenden Ansprüche, die weiterhin mindestens ein Enzym zum Erhöhen der Abbaurate der blutstillenden Zusammensetzung umfasst.

11. Blutstillende Zusammensetzung gemäß einem beliebigen der vorhergehenden Ansprüche, die in der Form einer sprühbaren Zusammensetzung, eines Filmes oder eines Schaums besteht.

12. Verwendung von mindestens einer biologisch abbaubaren, nicht-inflammatorischen Nukleinsäure zur Herstellung einer blutstillenden Zusammensetzung gemäß einem der Ansprüche 1 bis 11, die zum Fördern der Blutgerinnung einer Gewebestelle in einem Tierobjekt oder zum Fördern der blutgerinnenden Eigenschaften eines Substrats geeignet ist.

13. Verwendung gemäß Anspruch 12, wobei die blutstillende Zusammensetzung in der Form zum Tröpfeln, Ausstreichen, direkten Handhaben oder Aufsprühen auf eine Gewebeoberfläche bereitgestellt wird.

14. Verwendung gemäß Anspruch 12, wobei die blutstillende Zusammensetzung in der Form eines Filmes oder eines Schaumes bereitgestellt wird.

15. Biologisch abbaubare, nicht-inflammatorische Nukleinsäure zur Verwendung bei der Bildung eines biologisch abbaubaren blutstillenden Grundgerüstes aus Nukleinsäuren an einer Gewebestelle zum Herbeiführen der Blutstillung durch Anschwellen bei Kontakt mit Blut und Anziehen von Blutplättchen, Gerinnungsfaktoren und anderen Molekülen und/oder Zellen zur Oberfläche der Zusammensetzung, um die Blutgerinnung auszulösen.

## Revendications

1. Composition hémostatique comprenant une matrice d'au moins un acide nucléique biodégradable et non-inflammatoire pour induire l'hémostase par gonflage en contact avec le sang et en aspirant les plaquettes, facteurs de coagulation et autres molécules et/ou cellules à la surface de la composition pour initier la coagulation.

2. Composition hémostatique selon la revendication 1, dans laquelle le au moins un acide nucléique est synthétique.

3. Composition hémostatique selon la revendication 1, dans laquelle le au moins un acide nucléique est obtenu d'au moins une plante.

4. Composition hémostatique selon la revendication 3, dans laquelle la au moins une plante est sélectionnée parmi les oignons, le brocoli et les tomates.

5. Composition hémostatique selon la revendication 1, dans laquelle le au moins un acide nucléique est obtenu d'au moins un animal.

6. Composition hémostatique selon la revendication 5, dans laquelle le au moins un animal est sélectionné parmi l'humain, bovin, équin, murin et caprin.

7. Composition hémostatique selon l'une quelconque des revendications précédentes, où le au moins un acide nucléique est sélectionné parmi les ribonucléotides et les désoxyribonucléotides.

8. Composition hémostatique selon l'une quelconque des revendications précédentes, dans laquelle la composition a été stérilisée.

9. Composition hémostatique selon l'une quelconque des revendications précédentes, qui comprend en outre au moins un agent médicinal.

10. Composition hémostatique selon l'une quelconque des revendications précédentes, qui comprend en outre au moins une enzyme pour augmenter la vitesse de dégradation de la composition hémostatique.

11. Composition hémostatique selon l'une quelconque des revendications précédentes, qui est présente sous la forme d'une composition pulvérisable, d'un film ou d'une mousse.

12. Utilisation d'au moins un acide nucléique biodégradable, non inflammatoire, pour la fabrication d'une composition hémostatique selon l'une quelconque des revendications 1 à 11 apte à promouvoir l'hémostase d'un site de tissu dans un sujet animal ou pour promouvoir les propriétés hémostatiques d'un substrat.

13. Utilisation selon la revendication 12, dans laquelle la composition hémostatique est réalisée sous une forme pour le dégouttement, brossage, manipulation ou pulvérisation directe sur une surface de tissu.

14. Utilisation selon la revendication 12, dans laquelle la composition hémostatique est réalisée sous la forme d'un film ou d'une mousse.

15. Acide nucléique biodégradable, non-inflammatoire pour utilisation dans la formation d'une matrice hémostatique biodégradable d'acides nucléiques dans un site de tissu pour induire l'hémostase par gonflement en contact avec le sang, et pour aspirer les plaquettes, facteurs de coagulation et autres molécules et/ou cellules à la surface de la composition pour initier la coagulation.
